(19) Europäisches Patentamt
European Patent Office
Office européen des brevets

(11) Veröffentlichungsnummer : **0 057 668**
**B1**

(12) # EUROPÄISCHE PATENTSCHRIFT

(45) Veröffentlichungstag der Patentschrift : **09.01.85**

(51) Int. Cl.⁴ : **C 07 F 9/38**, C 08 K 5/53

(21) Anmeldenummer : **82810036.2**

(22) Anmeldetag : **28.01.82**

(54) **Phosphonsäuresalze, deren Herstellung und Verwendung zum Flammfestmachen von organischem Fasermaterial.**

(30) Priorität : **03.02.81 CH 700/81**

(43) Veröffentlichungstag der Anmeldung : **11.08.82 Patentblatt 82/32**

(45) Bekanntmachung des Hinweises auf die Patenterteilung : **09.01.85 Patentblatt 85/02**

(84) Benannte Vertragsstaaten : **BE CH DE FR GB IT LI**

(56) Entgegenhaltungen :
**DE-A- 2 827 867**
**DE-A- 2 903 928**
**US-A- 4 308 197**
**Chemical Abstracts, Band 93, No. 20, 17. November 1980 Columbus, Ohio, USA MATSUSHITA ELECTRIC INDUSTRIAL "Fire-resistant acetal resin compositions" page 39, column 1, abstract no. 187314r**

(73) Patentinhaber : **CIBA-GEIGY AG**
**Postfach**
**CH-4002 Basel (CH)**

(72) Erfinder : **Nachbur, Hermann**
**Mischelistrasse 21**
**CH-4153 Reinach (CH)**
Erfinder : **Guth, Christian**
**In den Klostermatten 26**
**CH-4052 Basel (CH)**

**EP 0 057 668 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstände der vorliegenden Erfindung sind neue Phosphonsäuresalze, deren Herstellungsverfahren und deren Verwendung als Flammschutzmittel für organisches Fasermaterial.

Aus DE-A-2 827 867 sind Gemische aus Kohlenstoffverbindungen des Phosphors, z. B. Phospinsäuren oder Phosphonsäuren, deren Salze oder Alkylderivate, und aus Stickstoffverbindungen, z. B. Dicyandiamid und/oder Guanidin und beispielsweise auch ein Guanidin-Addukt der Methyl-äthyl-phosphinsäure bekannt, die als Flammschutzmittel für Kunststoffe, insbesondere Formmassen, Verwendung finden.

Im Gegensatz hierzu werden gemäss vorliegender Erfindung neue Phosphonsäuresalze zur Verfügung gestellt, die aus Aethan- oder Methanphosphonsäure, Dicyandiamid und/oder Guanidin und gegebenenfalls Ammoniak hergestellt werden und als Flammschutzmittel für organische Fasermaterialien, insbesondere Textilien, Verwendung finden.

Die erfindungsgemässen Phosphonsäuresalze entsprechen demgemäss der Formel

(1) 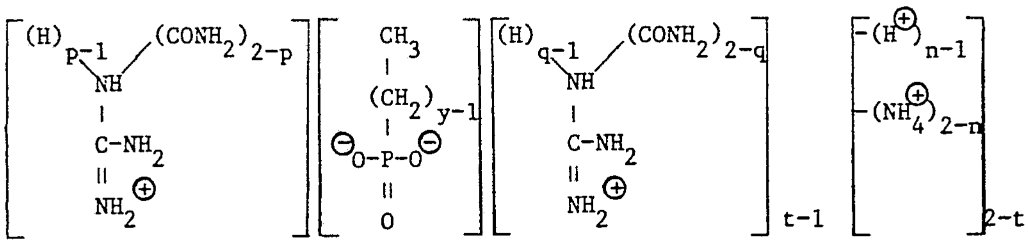

worin n, p, q, t und y je 1 oder 2 sind.

Wenn t in Formel (1) 2 ist, entsprechen die Phosphonsäuresalze der Formel

(2) 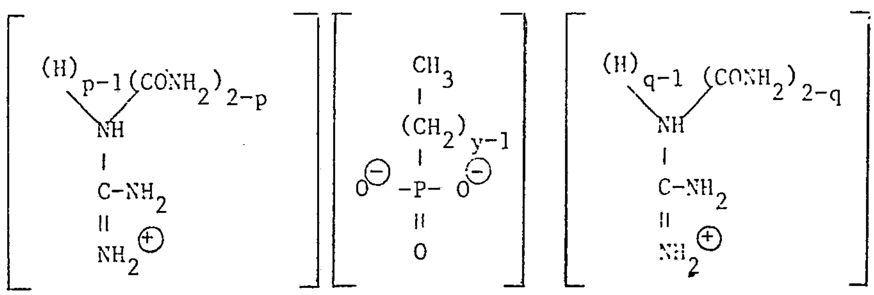

worin p, q und y je 1 oder 2 sind.

Falls p und q in Formel (2) von einander verschieden sind, d. h. falls p für 1 und q für 2 bzw. p für 2 und q für 1 stehen, entsprechen die Phosphonsäuresalze der Formel

(3) 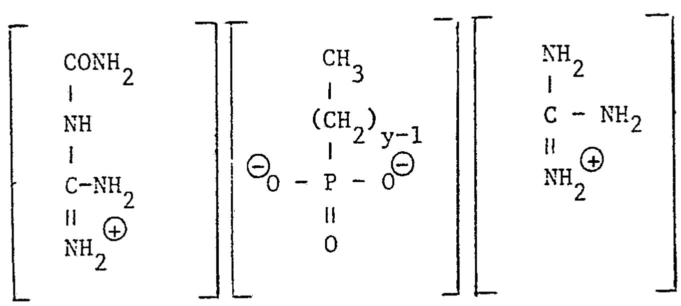

worin y 1 oder 2, jedoch vorzugsweise 1 ist.

Bevorzugt ist somit das Phosphonsäuresalz der Formel

(4) 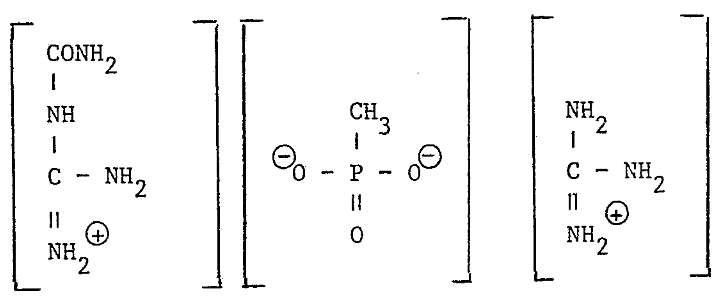

Falls hingegen p und q in Formel (2) die gleichen Bedeutungen haben, entsprechen die Phosphonsäuresalze, sofern p und q 2 sind, der Formel

(5) 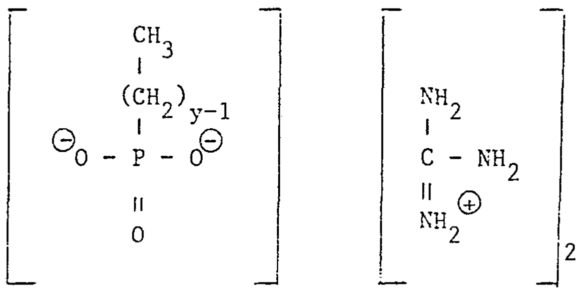

worin y 1 oder 2, jedoch vorzugsweise 1 ist.

Bevorzugt ist somit das Phosphonsäuresalz der Formel

(6) 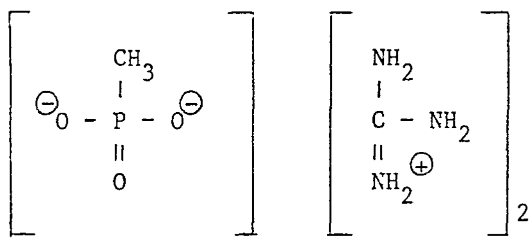

Falls p und q in Formel (2) die gleichen Bedeutungen haben und p und q 1 sind, entsprechen die Phosphonsäuresalze der Formel

(7) 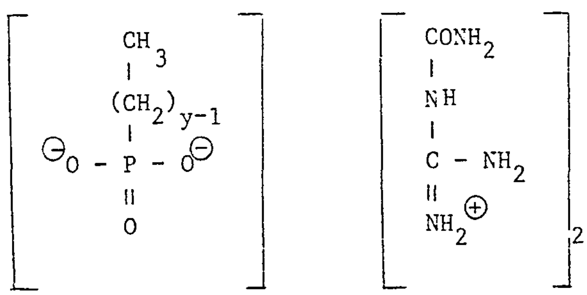

worin y 1 oder 2, jedoch vorzugsweise 1 ist.

Bevorzugt ist somit das Phosphonsäuresalz der Formel

(8) 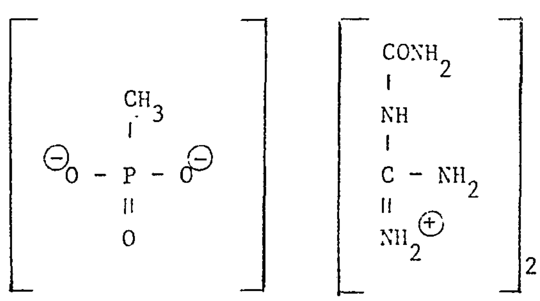

Falls in Formel (1) t für 2 steht und p und q die gleichen Bedeutungen haben, bedeuten p und q vorzugsweise 2. Somit sind die Phosphonsäuresalze der Formel (5) und vor allem (6) den Phosphonsäuresalzen der Formel (7) und (8) gegenüber bevorzugt. Von den Phosphonsäuresalzen der Formel (1), in welcher t 2 ist, sind somit die Salze der Formel (3) und vor allem (4), sowie die Salze der Formel (5) und vor allem (6) von besonderem Interesse.

Falls t in Formel (1) 1 ist, entsprechen die Phosphonsäuresalze der Formel

(9) 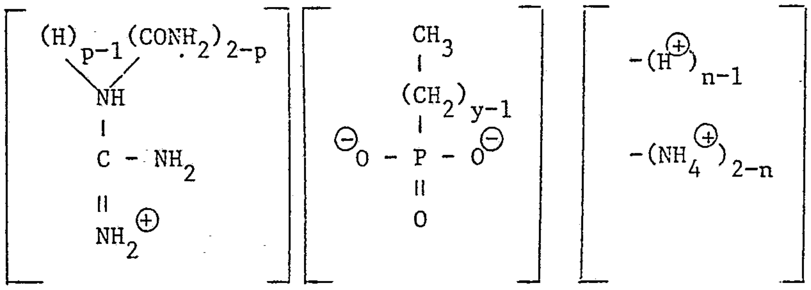

worin n, p und y je 1 oder 2 sind.

Falls p in Formel (9) 2 ist, entsprechen die Phosphonsäuresalze der Formel

(10) 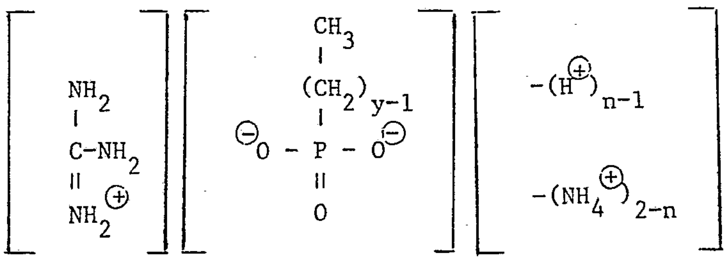

worin n und y je 1 oder 2 sind.

In Formel (10) steht y vorzugsweise für 1. Somit entsprechen bevorzugte Phosphonsäuresalze der Formel

(11) 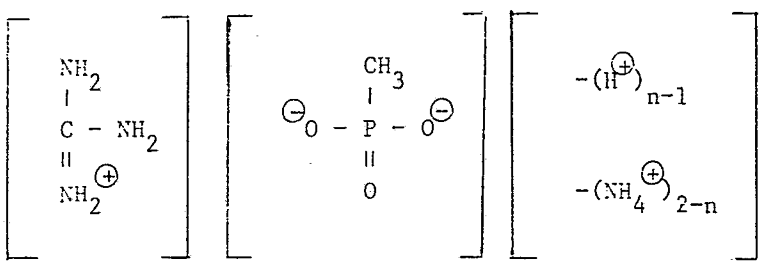

worin n 1 oder 2 ist.

Wenn n in Formel (11) für 1 steht, entspricht das in Frage kommende Phosphonsaüresalz der Formel

(12) 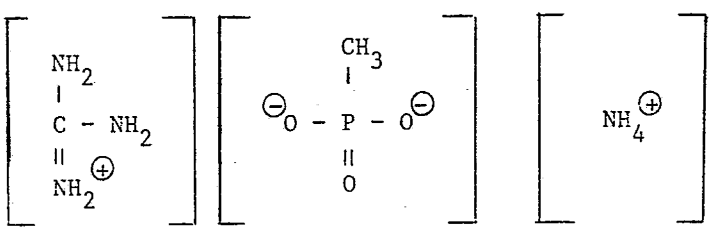

Wenn hingegen n in Formel (11) für 2 steht, entspricht das in Frage kommende Phosphonsäuresalz der Formel

(13) 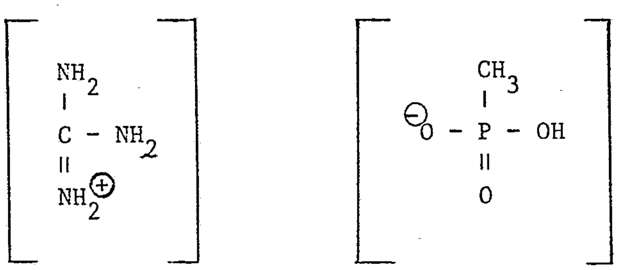

Falls p in Formel (9) 1 ist, entsprechen die Phosphonsäuresalze der Formel

(14) 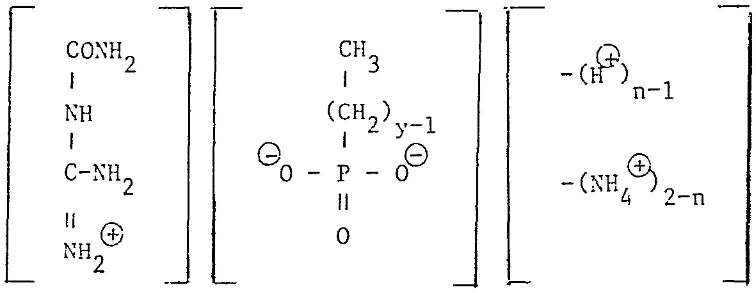

worin n und y je 1 oder 2 sind.

In Formel (14) steht y vorzugsweise für 1. Somit entsprechen bevorzugte Phosphonsäuresalze der Formel

(15) 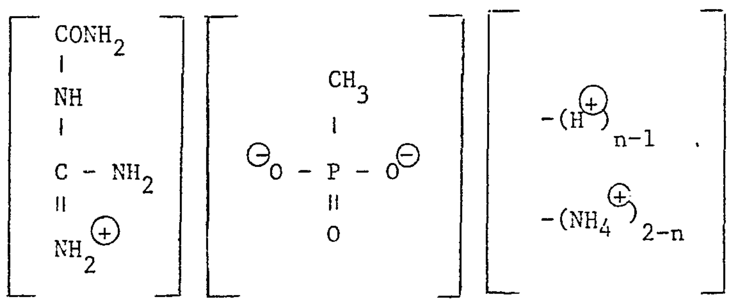

worin n 1 oder 2 ist.

Wenn n in Formel (15) für 1 steht, entspricht das in Frage kommende Phosphonsäuresalz der Formel

(16) 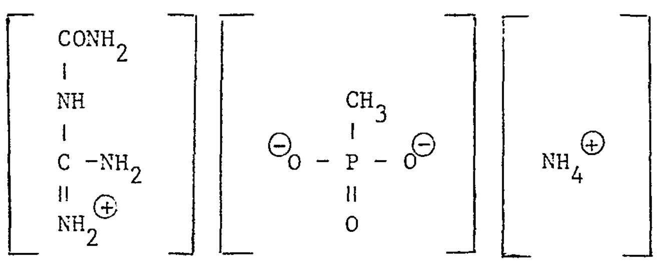

Wenn hingegen n in Formel (15) für 2 steht, entspricht das in Frage kommende Phosphonsäuresalz der Formel

(17) 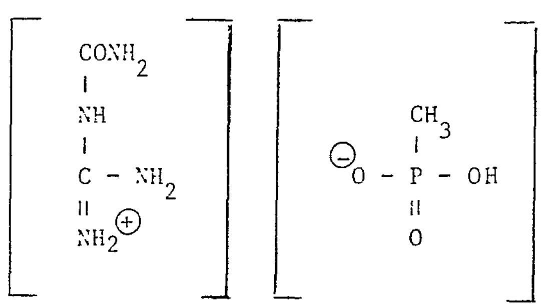

Falls in Formel (1) t für 1 steht, bedeuten n vorzugsweise 2 und p vorzugsweise 1. Somit sind von den Phosphonsäuresalzen der Formel (1), in welcher t 1 ist, die Salze der Formeln (14), (15), (16) und (17) den Salzen der Formeln (10), (11), (12) und (13) gegenüber bevorzugt, wobei das Salz der Formel (13) und insbesondere das Salz der Formel (17) im Vordergrund des Interesses stehen.

Die erfindungsgemässen Phosphonsäuresalze werden nach an sich bekannten Methoden durch Umsetzen der entsprechenden phosphorhaltigen Säuren mit den entsprechenden stickstoffhaltigen

Basen hergestellt. Das Verfahren zur Herstellung der Phosphonsäuresalze der Formel (1) ist dadurch gekennzeichnet, dass man 1 Mol Phosphonsäure der Formel

(18)
$$CH_3-(CH_2)_{y-1}-\overset{\overset{\displaystyle O}{\|}}{\underset{\underset{\displaystyle OH}{|}}{P}}-OH$$

worin y 1 oder 2 ist, mit 1 Mol einer Stickstoffverbindung der Formel

(19)
$$HN=\overset{\overset{\displaystyle NH_2}{|}}{C}-NH(-H)_{p-1}(-CN)_{2-p}$$

worin p 1 oder 2 ist und 1 Mol einer Stickstoffverbindung der Formel

(20)
$$HN=\overset{\overset{\displaystyle NH_2}{|}}{C}-NH(-H)_{q-1}(-CN)_{2-q}$$

worin q 1 oder 2 ist, sofern t in Formel (1) für 2 steht, oder mit 1 Mol einer Stickstoffverbindung der Formel (19) und gegebenenfalls 1 Mol Ammoniak, sofern t in Formel (1) für 1 steht, in wässrigem Medium gegebenenfalls bei erhöhter Temperatur umsetzt, wobei die Stickstoffverbindung als freies Guanidin oder vorzugsweise als Guanidincarbonat vorliegt, sofern p und q in Formel (19) und (20) für 2 stehen und die Umsetzung unter Anlagerung von 1 Mol Wasser erfolgt, sofern p und q für 1 stehen oder unter Abspaltung von 1 Mol Kohlendioxyd erfolgt, sofern Guanidincarbonat eingesetzt wird.

Bei der Ausgangsverbindung der Formel (18) handelt es sich je nach Bedeutung von y um Aethan- oder vorzugsweise Methanphosphonsäure, die z. B. in J. Am. Chem. Soc. *75,* 3379 ff (1953) beschrieben ist. Bei den Ausgangsverbindungen der Formeln (19) und (20) handelt es sich um Dicyandiamid, sofern p und q 1 sind und um Guanidin, sofern p und q 2 sind.

Sofern Dicyandiamid eingesetzt wird, erfolgt bei der Ulmsetzung wie bereits angedeutet eine Wasseranlagerung. Die Umsetzung mit Dicyandiamid wird vorzugsweise bei erhöhter Temperatur, z. B. 60 bis 100 °C, vorzugsweise 85 bis 95 °C durchgeführt. Es kommen jedoch auch höhere Umsetzungstemperaturen von z. B. 100 bis 130 °C in Frage, sofern die Umsetzung mit Dicyandiamid unter Druck durchgeführt wird.

Bei den Umsetzungen mit Guanidin wird vorzugsweise dessen Salz, nämlich das Guanidincarbonat eingesetzt, da Guanidinsalze leichter zugänglich sind als freies Guanidin. Als Salz kommt aus praktischen Gründen nur Guanidincarbonat in Betracht, weil die entsprechende schwache Säure, d. h. Kohlensäure, der Umsetzungsreaktion nicht teilnimmt, sondern unter den angewandten Arbeitsbedingungen als Kohlendioxyd dem Reaktionsgemisch entzogen wird, im Gegensatz zu den freien Säuren der andersartigen Guanidinsalzen wie Guanidin-hydrochlorid oder Guanidinacetat. Bei Verwendung von Guanidincarbonat wird bei den vorstehend angegebenen Molverhältnissen der Umstand berücksichtigt, dass pro Mol Guanidincarbonat 2 Mol freies Guanidin vorhanden sind. Die Umsetzungen mit Guanidin oder dessen Carbonat werden gegebenenfalls bei erhöhter Temperatur, z. B. 20 bis 100 °C, vorzugsweise 20 bis 60 °C und insbesondere 40 bis 60 °C durchgeführt. Bei Verwendung von Guanidincarbonat ist die Temperatur von 40 bis 60 °C besonders angezeigt und die Kohlendioxydentwicklung zu beschleunigen. Unter vermindertem Druck kann die Entfernung des Kohlendioxyds aus dem Reaktionsgemisch vervollständigt, bzw. beschleunigt werden, wobei in diesem Falle Temperaturen von 20 bis 40 °C auch in Betracht kommen.

Die Umsetzung mit Ammoniak findet nur dann statt, wenn n und t in Formel (1) beide für 1 stehen. In diesem Fall wird das Ammoniak vorzugsweise in Form einer wässrigen Lösung eingesetzt. Die fakultative Umsetzungen mit Ammoniak werden vorzugsweise bei 20 °C oder bei leicht erhöhten Temperaturen von 20 bis 40 °C durchgeführt.

Die Reihenfolge, mit welcher die Ausgangskomponenten umgesetzt werden, sind von massgebender Bedeutung. Bei der Umsetzung mit Dicyaniamid allein wird die Phosphonsäure vorgelegt und anschliessend mit Dicyandiamid umgesetzt, während bei der Umsetzung mit Guanidin allein vorzugsweise Guanidincarbonat vorgelegt und anschliessend mit der Phosphonsäure umgesetzt wird. Werden sowohl Dicyandiamid als auch Guanidin eingesetzt, so wird die Phosphonsäure vorgelegt, dann zuerst mit Dicyandiamid und zuletzt mit Guanidin oder vorzugsweise Guanidincarbonat umgesetzt. Werden die fakultativen Umsetzungen mit Ammoniak durchgeführt, so wird Ammoniak vorzugsweise zuletzt dem Reaktionsgemisch zugegeben, sofern Dicyandiamid eingesetzt wird, während bei Verwendung von

Guanidin die Phosphonsäure zuerst mit Ammoniak und zuletzt mit Guanidin, vorzugsweise Guanidincarbonat umgesetzt wird.

Zur Herstellung der Phosphonsäuresalze der Formel (2) wird so verfahren, dass man 1 Mol Aethan- oder Methanphosphonsäure mit 2 Mol Dicyandiamid oder 1 Mol Guanidincarbonat, sofern p und q in Formel (2) die gleichen Bedeutungen haben, oder mit 1 Mol Dicyandiamid und 0,5 Mol Guanidincarbonat, sofern p und q in Formel (2) voneinander verschieden sind, umsetzt.

Zur Herstellung des Phosphonsäuresalzes der Formeln (3) und (4) wird 1 Mol Aethan- oder vorzugsweise Methanphosphonsäure mit 1 Mol Dicyandiamid bei 60 bis 100 °C und anschliessend mit 0,5 Mol Guanidincarbonat bei 20 bis 60 °C umgesetzt, zur Herstellung der Phosphonsäuresalze der Formeln (5) und (6) wird 1 Mol Guanidincarbonat mit 1 Mol Aethan- oder vorzugsweise Methanphosphonsäure bei 20 bis 60 °C umgesetzt und zur Herstellung der Phosphonsäuresalze der Formeln (7) und (8) wird 1 Mol Aethan- oder vorzugsweise Methanphosphonsäure mit 2 Mol Dicyandiamid bei 60 bis 100 °C umgesetzt.

Zur Herstellung der Phosphonsäuresalze der Formel (9) wird so verfahren, dass man 1 Mol Aethan- oder Methanphosphonsäure mit 1 Mol Dicyandiamid, sofern p in Formel (9) 1 ist, oder 0,5 Mol Guanidincarbonat, sofern p in Formel (9) 2 ist, und 1 Mol einer wässrigen Ammoniaklösung, sofern n in Formel (9) 1 ist, umsetzt.

Zur Herstellung der Phosphonsäuresalze der Formeln (10) und (11) wird unter Einsatz von Guanidincarbonat analog verfahren.

Zur Herstellung des Phosphonsäuresalzes der Formel (12) wird 1 Mol Methanphosphonsäure mit 1 Mol einer wässrigen Ammoniaklösung und anschliessend 0,5 Mol Guanidincarbonat und zur Herstellung des Phosphonsäuresalzes der Formel (13) 0,5 Mol Guanidincarbonat mit 1 Mol Methanphosphonsäure bei 20 bis 60 °C umgesetzt.

Zur Herstellung der Phosphonsäuresalze der Formeln (14) und (15) wird unter Einsatz von Dicyandiamid analog verfahren.

Zur Herstellung des Phosphonsäuresalzes der Formel (16) wird 1 Mol Methanphosphonsäure mit 1 Mol Dicyandiamid bei 60 bis 100 °C und anschliessend mit 1 Mol einer wässrigen Ammoniaklösung bei 20 bis 60 °C umgesetzt. Zur Herstellung des Phosphonsäuresalzes der Formel (17) wird die soeben beschrieben verfahren, jedoch ohne die Ammoniaklösung einzusetzen.

Bei der Applikation zum Flammfestmachen von organischem Fasermaterial, bei welchem die Phosphonsäuresalze der Formel (1) als Flammschutzmittel verwendet werden, wird dieses Material mit der wässrigen Lösung mindestens eines Phosphonsäuresalzes der Formel (1) behandelt und getrocknet.

In diesem Verfahren wird die Behandlung des Fasermaterials im allgemeinen sowohl nach dem Aufsprüh- als auch insbesondere nach dem Foulardierverfahren durchgeführt. Ferner kommt auch z. B. das Tauch- oder vorzugsweise das Pflatschverfahren in Betracht.

Da die Phosphonsäuresalze der Formel (1) wasserlöslich sind, sind Zusätze von Hilfsmitteln zur Löslichmachung in den Applikationsbädern, -flotten oder -sprühlösungen in der Regel nicht notwendig. Es können dagegen vorteilhaft übliche Weichgriffmittel oder Tenside mitverwendet werden.

Im bevorzugten Foulardierverfahren werden die Phosphonsäuresalzlösungen mit einer Flottenaufnahme von z. B. 60 bis 110, vorzugsweise 60 bis 100, insbesondere 65 bis 80 Gewichtsprozent auf das auszurüstende Fasermaterial aufgebracht und das imprägnierte Material wird anschliessed in der Regel bei Temperaturen von 60 bis 200 °C, vorzugsweise jedoch bei höchstens 150 °C, z. B. 60 bis 150 °C und insbesondere 120 bis 150 °C getrocknet.

Das erfindungsgemässe Verfahren eignet sich zum Flammfestmachen von organischen Fasermaterialien, u. a. Holz, vorzugsweise Papier, z. B. Tapeten, oder insbesondere Textilien in beliebiger Verarbeitungsstufe, wie Fäden, Garne, Spulen, Vliese, Gewirke, Gewebe oder fertige Kleidungsstücke, oder Dekorationsmaterialien wie Teppiche, Möbelbezüge, Vorhänge oder stoffbeschichtete Tapeten.

Das auszurüstende organische Fasermaterial kann natürlicher oder synthetischer Herkunft sein oder aus Mischungen von natürlichen und synthetischen Fasern bestehen. Als natürliche Fasern kommen vor allem keratin- oder cellulosehaltige Fasern, inklusive Fasern aus regenerierter Cellulose, wie Leinen-, Hanf-, Sisal-, Ramie-, vorzugsweise Woll-, Baumwoll- und/oder Kunstseide-, Zellwolle- oder Viskosefasern in Frage.

Neben reinen Cellulosefasern kommen deren Gemische mit synthetischen Fasern in Betracht, wobei der Celluloseanteil vorzugsweise 20 bis 80 Gewichtsprozent der Mischung beträgt. Als synthetische Fasern kommen z. B. Polyester, vorzugsweise Acrylnitrilmischpolymerisate oder insbesondere Polyacrylnitril in Frage. Obwohl weniger bevorzugt kommen auch Fasern aus Celluloseacetat, z. B. Cellulose-2 1/2-acetat und Cellulosetriacetat und Fasern aus vernetzten Polyvinylalkoholen, z. B. Acetate oder Ketale von Polyvinylalkoholen in Betracht.

Im Vordergrund des Interesses stehen neben Cellulosefasern und deren Mischungen mit synthetischen Fasern indessen rein synthetische Fasermaterialien, insbesondere solche aus Polyester oder vor allem Polyacrylnitril oder Acrylnitrilmischpolymerisaten. Tapeten aus Polyacrylnitril lassen sich erfindungsgemäss besonders gut flammfest ausrüsten.

Solche Polyesterfasern leiten sich vor allem von der Terephthalsäure ab, z. B. von Poly-(äthylenglycolterephthalat) oder Poly(1,4-cyclohexyldimethylenterephthalat) ab.

Bei den Acrylnitrilmischpolymerisaten beträgt der Acrylnitrilanteil zweckmässig mindestens 50, vorzugsweise mindestens 85 Gewichtsprozent des Mischpolymerisats. Hierbei handelt es sich vor allem

um Mischpolymerisate, zu deren Herstellung andere Vinylverbindungen, z. B. Vinylchlorid, Vinylidenchlorid, Methylacrylate, Acrylamid oder Styrolsulfonsäuren, als Comonomere eingesetzt worden sind.

Die wässrigen Lösungen, mit denen diese Fasermaterialien behandelt werden, enthalten in der Regel 25 bis 500 g/l der Phosphonsäuresalze der Formel (1) und gegebenenfalls übliche Zusätze wie die vorstehend erwähnten Weichgriffmittel oder Tenside.

Insbesondere beim Behandeln von rein synthetischen Fasermaterialien aus z. B. Polyester nach dem bevorzugten Foulardierverfahren genügen oft Badkonzentrationen von 25 bis 100 g/l, vor allem wenn dabei Ammoniumsalze eingesetzt werden. Vorzugsweise wird aber mit Badkonzentrationen im Foulardierverfahren von 200 bis 450 g/l gearbeitet, vor allem beim Flammfestausrüsten von Polyacrylnitrilfasern.

Der pH-Wert der erfindungsgemäss verwendeten Lösungen beträgt im allgemeinen 3 bis 9.

Die notwendigen Auflagen an Verbindung der Formel (1) zur Erzielung eines genügenden Flammschutzeffektes sind je nach Faser- und Materialart verschieden und bewegen sich in der Regel zwischen 2 und 40 %, bezogen auf das Fasergewicht.

Nach dem erfindungsgemässen Verfahren werden keine permanenten Flammschutzeffekte erzielt, darum sollen die behandelten Fasermaterialien auch keiner Nachwäsche unterworfen werden.

Das erfindungsgemässe Verfahren zeichnet sich vor allem dadurch aus, dass sich damit die verschiedensten Substrate wirksam flammfest ausrüsten lassen.

Die Lichtechtheit von gefärbten oder aufgehellten Fasermaterialien werden durch die erfindungsgemässen Ausrüstungen praktisch nicht beeinflusst. Auch die gute Kompatibilität der erfindungsgemäss verwendeten Phosphonsäuresalze mit den meisten Textilveredlungsmitteln wie wasser- und ölabweisende Mittel, Steif- und Weichgriffmittel sind besonders vorteilhaft.

Die geringen notwendigen Auflagemengen beim Flammfestmachen von Polyesterfasern sind ein weiterer Vorteil des erfindungsgemässen Verfahrens.

Der wesentlichste Vorteil der vorliegenden Erfindung besteht aber darin, dass die Fasermaterialien nicht nur bei üblichen Trocknungstemperaturen bis zu 100 °C, sondern auch bei höheren Temperaturen, z. B. 100 bis 200 °C, insbesondere 120 bis 150 °C getrocknet werden können, ohne dass die mechanischen Eigenschaften der so getrockneten Fasermaterialien wie der Griff, die Hygroskopizität und die Reissfestigkeit wesentlich verschlechtert werden. Insbesondere neigen die erfindungsgemäss behandelten Fasermaterialien bei hohen Trocknungstemperaturen weniger zur Vergilbung als dies der Fall ist für nach bekannter Weise flammfest ausgerüstete Fasermaterialien. Beim Trocknen bei erhöhter Temperatur kann die Trocknungszeit desto kürzer gehalten werden, je höher die Temperatur ist. Bei 200 °C genügt in der Regel eine Trocknungsdauer von 1/2 bis 1 Minute. Bei 140 bis 160 °C werden in der Regel 2 bis 10 Minuten benötigt. Diese verkürzte Trocknungszeit erlaubt durch die Zeiteinsparung eine höhere Kapazität in den Ausrüstbetrieben. Die Trocknung bei erhöhter Temperatur, auch als sogenannte « Uebertrocknung » bezeichnet, kann in der Praxis mit Düsentrocknern üblicher Bauart durchgeführt werden.

Bei Uebertrocknung neigen vor allem cellulosehaltige Fasermaterialien stark zu vergilben und ihre textilmechanische Eigenschaften nachteilig zu verändern. Bei der Applikation der erfindungsgemässen Phosphonsäuresalze als Flammschutzmittel kann im Gegensatz zu bisher verwendeten Flammschutzmitteln, insbesondere bekannten Phosphonsäuresalzen, die Vergilbung und die Verschlechterung der textilmechanischen Eigenschaften selbst bei der Uebertrocknung von vor allem cellulosehaltigen Fasermaterialien unerwarteterweise weitgehend eliminiert werden.

Demgemäss dürfen im erfindungsgemässen Applikationsverfahren Cellulosefasern nach dem Foulardierverfahren mit einer wässrigen Lösung der Phosphonsäuresalze der Formel (1) imprägniert und anschliessend bei 120 bis 150 °C getrocknet werden.

Die Angabe der Prozente und Teile in den nachfolgenden Beispielen beziehen sich auf Gewichtseinheiten.

## Herstellungsbeispiele

### Beispiel 1

986 Teile einer 97,4 %igen Methanphosphonsäure (10 Mol) werden in 1 800 Teilen entionisiertem Wasser bei 20 °C gelöst und auf 90 °C geheizt. Innerhalb von 30 Minuten werden 840 Teile Dicyandiamid (10 Mol) bei 90 °C der vorgelegten Methanphosphonsäure unter zeitweiser Kühlung des Reaktionsgemisches zugegeben. Anschliessend wird das Reaktionsgemisch bei 90 °C während 90 Minuten gehalten und dann auf 20 °C gekühlt.

Man erhält 3 594 Teile einer wässrigen, farblosen, klaren Lösung, deren pH-Wert 3,0 und Gehalt an Phosphonsäuresalz der Formel (17) von 48,0 % (87,1 % der Theorie) und an nicht umgesetzter Methanphosphonsäure von 4,2 % (15,5 % der eingesetzten Menge) betragen, wobei die genannten Salz- und Säuregehalte aufgrund der Titration einer Probe der wässrigen Reaktionslösung mit einer wässrigen Natriumhydroxydlösung ermittelt werden.

Ein Teil der erhaltenen wässrigen Lösung wird unter vermindertem Druck bei höchstens 50 °C eingedampft und anschliessend unter vermindertem Druck bei 50 °C getrocknet.

200 Teile der so erhaltenen, getrockneten Rohsubstanz werden von der nicht umgesetzen Methanphosphonsäure gereinigt, indem die Rohsubstanz 3 mal mit je 200 Teilen Isopropanol bei 20 °C ausgewaschen und hierauf bei 50 °C unter vermindertem Druck getrocknet wird.

Die so gereinigte Rohsubstanz weist einen Schmelzpunkt von 151 °C und einen Gehalt an Phosphonsäuresalz der Formel (17) von 95,8 % auf.

Die gereinigte Rohsubstanz ist frei von nicht umgesetzter Methanphosphonsäure.

Zu Analysenzwecken werden 10 Teile der erhaltenen Rohsubstanz in 400 Teilen 95 %igem Aethanol umkristallisiert. Die umkristallisierte Reinsubstanz liegt als weisses Pulver vor und weist einen Schmelzpunkt von 163 °C auf.

Elementaranalyse der umkristallisierten Reinsubstanz :

| | | | | |
|---|---|---|---|---|
| Berechnet : | C 18,18 % | H 5,55 % | N 28,29 % | P 15,65 % |
| Gefunden : | C 18,2 % | H 5,4 % | N 28,3 % | P 15,4 % |

## Beispiel 2

Zu 207 Teilen der in Beispiel 1 erhaltenen, wässrigen Lösung mit einem Gehalt von 48 % an Phosphonsäuresalz der Formel (17) (0,5 Mol) und von 4,2 % an freie Methanphosphonsäure (0,09 Mol) werden innerhalb von 20 Minuten 46 Teile einer wässrigen, 25 %igen Ammoniaklösung (0,68 Mol) bei 20 °C unter zeitweiser Kühlung des Reaktionsgemisches zugegeben. Man erhält 253 Teile einer milchigtrüben, wässrigen Lösung, welche filtriert wird. Die nun klare Lösung weist einen Gehalt an Phosphonsäuresalz der Formel (16) von 42 % (100 % der Theorie) sowie einen Gehalt an Methanphosphonsäurediammoniumsalz von 4,6 % auf.

Ein Teil der erhaltenen wässrigen Lösung wird unter vermindertem Druck bei höchstens 50 °C eingedampft und anschliessend unter vermindertem Druck bei 50 °C getrocknet.

Die so erhaltenen Rohsubstanz schmilzt bei 90 bis 125 °C unter Zersetzung (Ammoniakentwicklung) und weist folgende Elementaranalyse auf :

| | | | | |
|---|---|---|---|---|
| Berechnet : | C 16,75 % | H 6,56 % | N 32,55 % | P 14,40 % |
| Gefunden : | C 16,6 % | H 6,4 % | N 29,7 % | P 14,1 % |

## Beispiel 3

Zu 413 Teilen der in Beispiel 1 erhaltenen, wässrigen Lösung mit einem Gehalt von 48 % an Phosphonsäuresalz der Formel (17) (1 Mol) und 4,2 % an freie Methanphosphonsäure (0,18 Mol) werden innerhalb von 30 Minuten eine Lösung von 124 Teilen eines 99 %igen Guanidincarbonates (0,68 Mol) in 2 150 Teilen entionisiertem Wasser bei 50 °C zugegeben. Bei dieser Temperatur wird das Reaktionsgemisch während 30 Minuten gehalten bis die $CO_2$-Entwicklung beendet ist. Anschliessend wird das Reaktionsgemisch auf 20 °C gekühlt. Man erhält 2 656 Teile einer farblosen, klaren, wässrigen Lösung, welche einen Gehalt an Phosphonsäuresalz der Formel (4) von 9,68 % (100 % der Theorie), sowie einen Gehalt an Phosphonsäuresalz der Formel (6) von 1,45 % aufweist.

Ein Teil der erhaltenen wässrigen Lösung wird unter vermindertem Druck bei höchstens 50 °C eingedampft und anschliessend unter vermindertem Druck bei 50 °C getrocknet.

10 Teile der so erhaltenen Rohsubstanz werden in 100 Teilen Aethanol und 35 Teilen Wasser umkristallisiert. Man erhält als umkristallisierte Substanz das Phosphorsäuresalz der Formel (4), das unter Zersetzung bei 298 bis 300 °C schmilzt und folgende Elementaranalyse aufweist :

| | | | | |
|---|---|---|---|---|
| Berechnet : | C 17,44 % | H 6,62 % | N 35,59 % | P 11,24 % |
| Gefunden : | C 17,9 % | H 7,1 % | N 35,6 % | P 11,2 % |

## Beispiel 4

In eine Lösung von 455 Teilen eines 99 %igen Guanidincarbonats (2,5 Mol) in 1 000 Teilen entionisiertem Wasser wird innerhalb von 15 Minuten eine Lösung von 512,5 Teilen einer 93,7 %igen Methanphosphonsäure (5 Mol) in 100 Teilen entionisiertem Wasser bei 20 °C zugegeben, wobei sich das Reaktionsgemisch auf 25 °C erwärmt. Hierauf wird das Reaktionsgemisch auf 50 °C geheizt, bei dieser Temperatur während 30 Minuten gehalten und unter vermindertem Druck bei höchstens 50 °C so konzentriert, dass man 1 550 Teile einer farblosen, klaren, wässrigen Lösung erhält, die einen Gehalt an Phosphonsäuresalz der Formel (13) von 50 % (100 % der Theorie) aufweist.

Ein Teil der erhaltenen wässrigen Lösung wird unter vermindertem Druck bei höchstens 50 °C eingedampft und anschliessend unter vermindertem Druck bei 50 °C getrocknet.

10 Teile der so erhaltenen Rohsubstanz werden in 200 Teilen absolutem Aethanol umkristallisiert. Die so umkristallisierte Substanz weist einen Schmelzpunkt von 125° bis 129 °C auf folgende Elementaranalyse auf :

Berechnet : C 15,49 % H 6,50 % N 27,09 % P 19,97 %
Gefunden : C 15,7 % H 6,6 % N 26,9 % P 19,9 %

Beispiel 5

In eine Lösung von 91 Teilen eines 99 %igen Guanidincarbonats (0,5 Mol) in 200 Teilen entionisiertem Wasser werden innerhalb von 20 Minuten eine Lösung von 51,25 Teilen einer 93,7 %igen Methanphosphonsäure (0,5 Mol) in 100 Teilen entionisiertem Wasser bei 20 °C zugegeben, wobei sich das Reaktionsgemisch auf 27 °C erwärmt. Hierauf wird das Reaktionsgemisch auf 50 °C geheizt, bei dieser Temperatur während 30 Minuten gehalten, unter vermindertem Druck bei höchstens 55 °C eingedampft und anschliessend unter vermindertem Druck bei 50 °C getrocknet. Man erhält 106 Teile des Phosphonsäuresalzes der Formel (6) (99 % der Theorie), welche als weisse, kristalline Substanz vorliegt, die bis 250 °C nicht schmilzt.

Elementaranalyse

Berechnet : C 16,83 % H 7,06 % N 39,24 % P 14,46 %
Gefunden : C 16,8 % H 6,8 % N 37,5 % P 14,7 %

Beispiel 6

In eine Lösung von 48,35 Teilen einer 99,3 %igen Methanphosphonsäure (0,5 Mol) in 200 Teilen entionisiertem Wasser werden innerhalb von 5 Minuten 34 Teile einer wässrigen 25 %igen Ammoniaklösung (0,5 Mol) zugegeben, wobei sich das Reaktionsgemisch auf 40 °C erwärmt.

Nach beendeter Ammoniakzugabe werden dem Reaktionsgemisch innerhalb von 5 Minuten eine Lösung von 45,5 Teilen eine 99 %igen Guanidincarbonats (0,25 Mol) in 200 Teilen entionisiertem Wasser zugegeben, wobei die Temperatur des Reaktionsgemisches auf 30 °C absinkt. Die so erhaltene, wässrige, opal-trübe Lösung wird unter vermindertem Druck bei höchstens 50 °C eingedampft und anschliessend unter vermindertem Druck bei 40 °C getrocknet.

Man erhält 83 Teile (96,4 % der Theorie) der Phosphonsäuresalzes der Formel (12), welches als weisse, kristalline Substanz vorliegt, die bei 137 °C unter Zersetzung schmilzt.

Elementaranalyse

Berechnet : C 13,96 % H 7,62 % N 32,54 % P 18,00 %
Gefunden : C 14,2 % H 6,9 % N 29,3 % P 18,8 %

Beispiel 7

59,15 Teile einer 97,4 %igen Methanphosphonsäure (0,6 Mol) werden in 292 Teilen entionisiertem Wasser bei 20 °C gelöst und auf 90 °C geheizt. Innerhalb von 15 Minuten werden 100,8 Teile Dicyandiamid (1,2 Mol) bei 90 °C der vorgelegten Methanphosphonsäure unter zweitweiser Kühlung des Reaktionsgemisches zugegeben. Anschliessend wird das Reaktionsgemisch bei 97 °C während 15 Stunden gehalten und dann auf 20 °C gekühlt. Nach Abfiltrieren eines während der Reaktion gebildeten, wasserunlöslichen Nebenproduktes (33 Teile Trockensubstanz) erhält man 390 Teile einer farblosen, klaren wässrigen Lösung, welche einen Gehalt von 38 % (82,3 % der Theorie) an Phosphonsäuresalz der Formel (8) aufweist.

Ein Teil der erhaltenen wässrigen Lösung wird unter vermindertem Druck bei höchstens 50 °C eingedampft und anschliessend unter vermindertem Druck bei 50 °C getrocknet.

Die so erhaltene Rohsubstanz schmilzt ab 155 °C unter Zersetzung und weist folgende Elementaranalyse auf :

Berechnet : C 20,00 % H 5,71 % N 37,33 % P 10,32 %
Gefunden : C 18,9 % H 5,9 % N 36,6 % P 10,4 %

Beispiel 8

135,8 Teile einer 81 %igen Aethanphosphonsäure (1 Mol) werden in 150 Teilen entionisiertem Wasser bei 20 °C gelöst und auf 85 °C geheizt. Innerhalb von 30 Minuten werden 84 Teile Dicyandiamid (1 Mol) bei 85 °C der vorgelegten Aethanphosphonsäure zugegeben. Nach beendigter Zugabe steigt die Temperatur des Reaktionsgemisches von selbst innert 10 Minuten auf 92 °C. Anschliessend wird das Reaktionsgemisch während 1 1/2 Stunden bei 85-90 °C gehalten und dann auf 20 °C gekühlt. Man erhält 372 Teile einer wässrigen fablosen, klaren Lösung, welche unter vermindertem Druck bei 60-65 °C eingedampft wird. Als Rohsubstanz erhält man 232 Teile (100 % der Theorie) eines partiell kristallisierten, 20 Teile Wasser enthaltenden weissen Produktes, welches in 300 Teilen 95 %igem Aethanol umkristallisiert wird. Man erhält 79,5 Teile der umkristallisierten Reinsubstanz der Formel (14), worin y und n 2 sind, die als kristallines, weisses Pulver vorliegt, bei 163 bis 164 °C unter Zersetzung schmilzt und die folgende Elementaranalyse aufweist :

| | | | | |
|---|---|---|---|---|
| Berechnet : | C 22,76 % | H 5,73 % | N 26,54 % | P 14,67 % |
| Gefunden : | C 22,8 % | H 6,0 % | N 26,4 % | P 14,6 % |

## Applikationsbeispiele

### Beispiel 9

Mit einer wässrigen Flotte der in der nachfolgenden Tabelle I angegebenen Zusammensetzung werden verschiedene Gewebe foulardiert und während 30 Min. bei 80 °C getrocknet. Nach der Trocknung wird die Prüfung des Flammschutzeffektes gemäss DOC FF 3-71 (Zündzeit 3 Sekunden) durchgeführt. Die Beurteilung der Griffbeeinflussung durch die Ausrüstung erfolgt nach der Trocknung der Gewebe. Der Griff wird aufgrund einer Notenskala von 0 bis 4 bewertet, wobei die beste Note 0 dem unbehandeltem Gewebe und die schlechteste Note 4 einem unerwünschtem steifen Griff entsprechen.

Alle Testergebnisse sind ebenfalls in der nachfolgenden Tabelle I zusammengestellt.

Tabelle I

| | Art und Flächengewichte der Gewebe | | | | | |
|---|---|---|---|---|---|---|
| | Baumwolle ($140\ g/m^2$) | | Polyacrylnitril ($138 g/m^2$) | | Polyester/Baumwolle 50:50 ($167\ g/m^2$) | |
| | behandelt | unbehandelt | behandelt | unbehandelt | behandelt | unbehandelt |
| g wässrige, 48%ige Lösung des Salzes der Formel (17) gemäss Beispiel 1 /1 Flotte | 166 | 0 | 702 | 0 | 694 | 0 |
| % Auflage an Salz der Formel (17) nach Trocknung | 6,6 | - | 37,0 | - | 22,1 | - |
| Flammfestigkeit<br>- Brennzeit (Sek.)<br>- Einreisslänge (cm) | <br>3<br>6 | brennt | <br>3<br>3,5 | brennt | <br>2<br>7 | brennt |
| Griff (Noten) | 0,5 | 0 | 2 | 0 | 2,5 | 0 |

Die Griffnoten zeigen, dass der Griff der behandelten Gewebe etwas voller ist, als der Griff der unbehandelten Gewebe.

### Beispiel 10

Mit einer wässrigen Flotte der in der nachfolgenden Tabelle II angegebenen Zusammensetzung wird ein Baumwollgewebe (Flächengewicht $140\ g/m^2$) foulardiert und während 5 Min. bei 150 °C in einem Düsentrockner getrocknet. Nach der Trocknung wird wie in Beispiel 9 angegeben die Prüfung des Flammschutzeffektes durchgeführt.

Anschliessend wird die Reissfestigkeit der Gewebe gemäss SNV 198 461 gemessen. Die Angaben entsprechen der prozentualen Reissfestigkeit bezogen aufs unbehandelte Material. Die Farbdifferenzwerte werden gemäss DIN 6174 D 65/10 ermittelt. Je Höher der Wert ist, desto stärker ist die Vergilbung. Alle Ergebnisse sind der nachfolgenden Tabelle II zu entnehmen.

Tabelle II

| | Behandeltes Baumwollgewebe | unbehandeltes Baumwollgewebe |
|---|---|---|
| g wässrige, 48%ige Lösung des Salzes der Formel (17) gemäss Beispiel 1 /1 Flotte | 208 | 0 |
| pH Wert der Flotte | 3,1 | - |
| % Auflage an Salz der Formel (17) nach Trocknung | 7,8 | 0 |
| Flammfestigkeit<br>- Brennzeit (Sek.)<br>- Einreisslänge (cm) | <br>2<br>8 | brennt |
| Reissfestigkeit %<br>- Kette<br>- Schuss | <br>92<br>88 | <br>100<br>100 |
| Farbdifferenzwerte | 3,5 | 0 |

Die Farbdifferenzwerte zeigen, dass das behandelte Gewebe im Vergleich zum unbehandelten nur unwesentlich vergilbt.

Beispiel 11

Mit einer wässrigen Flotte der in der nachfolgenden Tabelle III angegebenen Zusammensetzung wird ein Baumwollgewebe (Flächengewicht 140 g/m$^2$) foulardiert und während 5 Min. bei 150 °C getrocknet. Nach der Trocknung wird die Prüfung des Flammschutzeffektes, der Reissfestigkeit, der Farbdifferenz und des Griffes wie in den Beispielen 9 und 10 angegeben durchgeführt. Die Ergebnisse sind der nachfolgenden Tabelle III zu entnehmen.

(Siehe Tabelle III Seite 13 f.)

Tabelle III

| | behandeltes Baumwollgewebe | unbehandeltes Baumwollgewebe |
|---|---|---|
| g wässrige, 9,68%ige Lösung des Salzes der Formel (4) gemäss Beispiel 3 / 1 Flotte | 1000 | 0 |
| pH-Wert der Flotte | 6,2 | - |
| % Auflage an Salz der Formel (4) nach Trocknung | 7,8 | - |
| Flammfestigkeit<br>- Brennzeit (Sek.)<br>- Einreisslänge (cm) | <br>2<br>5,5 | brennt |
| Reissfestigkeit %<br>- Kette<br>- Schuss | <br>96<br>91 | <br>100<br>100 |
| Farbdifferenzwerte | 3,8 | 0 |
| Griff (Note) | 0,5 | 0 |

Aehnliche Ergebnisse werden mit den Salzen einer der Formeln (8), (12) und (16) erzielt.

Beispiel 12

Mit einer wässrigen Flotte der in der nachfolgenden Tabelle IV angegebenen Zusammensetzung werden verschiedene Gewebe foulardiert und während 30 Min. bei 80 °C getrocknet. Nach der Trocknung wird die Prüfung des Flammschutzeffektes wie in Beispiel 9 angegeben durchgeführt. Zudem werden nach der Trocknung der Weissgrad nach dem CIBA/GEIGY Weissmassstab (vgl. CIBA/GEIGY Rundschau 1973/1, Seiten 23 bis 24) (je tiefer der Weissgrad ist, desto stärker ist auch die Vergilbung) und nach der Trocknung und anschliessender 2-tägigen Lagerung der Gewebe bei 65 % relativer Feuchtigkeit die Hygroskopizität der behandelten Gewebe durch Gewichtszunahme gemessen. Die Hygroskopizität wird als % des ursprünglichen Gewebegewichts berechnet. Die Ergebnisse sind in nachfolgender Tabelle IV zusammengestellt.

(Siehe Tabelle IV Seite 14 f.)

Tabelle IV

| | Art und Flächengewicht der Gewebe | | | |
|---|---|---|---|---|
| | Polyacrylnitril (138 g/m$^2$) | | Polyester/Baumwolle 50 : 50 (167 g/m$^2$) | |
| | behandelt | unbehandelt | behandelt | unbehandelt |
| g Salz der Formel (6) gemäss Beispiel 5 / 1 Flotte | 270 | 0 | 300 | 0 |
| pH-Wert der Flotte | 8,8 | - | 8,8 | - |
| % Auflage an Salz der Formel (6) nach Trocknung | 26,8 | - | 24,9 | - |
| Flammfestigkeit<br>- Brennzeit (Sek.)<br>- Einreisslänge (cm) | <br>2<br>8 | brennt | <br>0<br>4 | brennt |
| Weissgrad | -44 | 15 | -33 | 68 |
| Hygroskopizität (%-Wasseraufnahme) | 1,4 | 0,9 | 6,6 | 7,2 |

Die Weissgrad-Messergebnisse zeigen, dass behandeltes im Vergleich mit unbehandeltem Gewebe nur unwesentlich stärker vergilbt.

Aehnliche Ergebnisse werden mit den Salzen einer der Formeln (4), (8), (12) und (16) erzielt.

Beispiel 13

Mit einer wässrigen Flotte der in der nachfolgenden Tabelle V angegebenen Zusammensetzung wird ein Baumwollgewebe (Flächengewicht 140 g/m$^2$) foulardiert und während 5 Min. bei 150 °C getrocknet. Nach der Trocknung wird die Prüfung des Flammschutzeffektes, der Reissfestigkeit, des Weissgrades und der Hygroskopizität wie in Beispielen 9, 10 und 12 angegeben durchgeführt. Die Ergebnisse sind in nachfolgender Tabelle V zusammengestellt.

(Siehe Tabelle V Seite 15 f.)

Tabelle V

| | Behandeltes Baumwollgewebe | | unbehandeltes Baumwollgewebe |
|---|---|---|---|
| g Salz der Formel (6) gemäss Beispiel 5 / 1 Flotte | 105 | 135 | 0 |
| pH-Wert der Flotte | 8,9 | 8,9 | - |
| % Auflage an Salz der Formel (6) nach Trocknung | 6,6 | 9,7 | - |
| Flammfestigkeit<br>- Brennzeit (Sek.)<br>- Einreisslänge (cm) | <br>1<br>7 | <br>2<br>6 | brennt |
| Reissfestigkeit %<br>- Kette<br>- Schuss | <br>105<br>92 | <br>98<br>97 | <br>100<br>100 |
| Weissgrad | - 9 | - 25 | 67 |
| Hygroskopizität (% Wasseraufnahme) | 6,8 | 6,8 | 7,4 |

Beispiel 14

Mit einer wässrigen Flotte der in der nachfolgenden Tabelle VI angegebenen Zusammensetzung wird ein Polyacrylnitrilgewebe (Flächengewicht 138 g/m$^2$) foulardiert und während 30 Min. bei 80 °C getrocknet. Nach der Trocknung wird die Prüfung des Flammschutzeffektes wie in Beispiel 9 angegeben durchgeführt. Die Ergebnisse sind der nachfolgenden Tabelle VI zu entnehmen.

Tabelle VI

| | Behandeltes Polyacrylnitrilgewebe | unbehandeltes Polyacrylnitrilgewebe |
|---|---|---|
| g wässrige, 50%ige Lösung des Salzes der Formel (13) gemäss Beispiel 4 / 1 Flotte | 500 | 0 |
| Flammfestigkeit<br>- Brennzeit (Sek.)<br>- Einreisslänge (cm) | <br>3<br>4 | brennt |

Beispiel 15

Mit einer wässrigen Flotte der in der nachfolgenden Tabelle VII angegebenen Zusammensetzung wird ein Baumwollgewebe (Flächengewicht 140 g/m²) foulardiert und während 5 Min. bei 150 °C getrocknet. Nach der Trocknung wird die Prüfung des Flammschutzeffektes und der Reissgestigkeit wie in Beispielen 9 und 10 angegeben durchgeführt. Die Ergebnisse sind der nachfolgenden Tabelle VII zu entnehmen.

Tabelle VII

| | Behandeltes Baumwollgewebe | unbehandeltes Baumwollgewebe |
|---|---|---|
| g wässrige, 50%ige Lösung des Salzes der Formel (13) gemäss Beispiel 4 / 1 Flotte | 250 | 0 |
| Flammfestigkeit<br>- Brennzeit (Sek.)<br>- Einreisslänge (cm) | <br>1<br>2,5 | brennt |
| Reissfestigkeit %<br>- Kette<br>- Schuss | <br>93<br>88 | <br>100<br>100 |

Beispiel 16

Mit einer wässrigen Flotte der in der nachfolgenden Tabelle VIII angegebenen Zusammensetzung wird ein Polyester/Baumwoll-Mischgewebe 50 : 50 (Flächengewicht : 167 g/m²) foulardiert und während 5 Minuten bei 150 °C in einem Düsentrockner getrocknet. Nach der Trocknung wird wie in Beispiel 9 angegeben die Prüfung des Flammschutzeffektes und wie in Beispiel 10 angegeben die Bestimmung der Reissfestigkeit und Farbdifferenzwerte durchgeführt. Die Ergebnisse sind der nachfolgenden Tabelle VIII zu entnehmen :

(Siehe Tabelle VIII Seite 17 f.)

Tabelle VIII

| | Behandeltes Polyester/ Baumwoll-Mischgewebe | Unbehandeltes Polyester/ Baumwoll-Mischgewebe |
|---|---|---|
| g Salz der Formel (7), worin y 2 ist, gemäss Beispiel 8 / 1 Flotte | 300 | 0 |
| pH-Wert der Flotte | 3,9 | - |
| % Auflage an Salz der Formel (7), worin y 2 ist, nach Trocknung | 20,2 | - |
| Flammfestigkeit<br>- Brennzeit (Sek.)<br>- Einreisslänge (cm) | <br>16<br>5 | <br>brennt |
| Reissfestigkeit %<br>- Kette<br>- Schuss | <br>95,7<br>77,4 | <br>100<br>100 |
| Farbdifferenzwerte | 2,4 | 0 |

**Ansprüche**

1. Phosphonsäuresalze der Formel

(1) 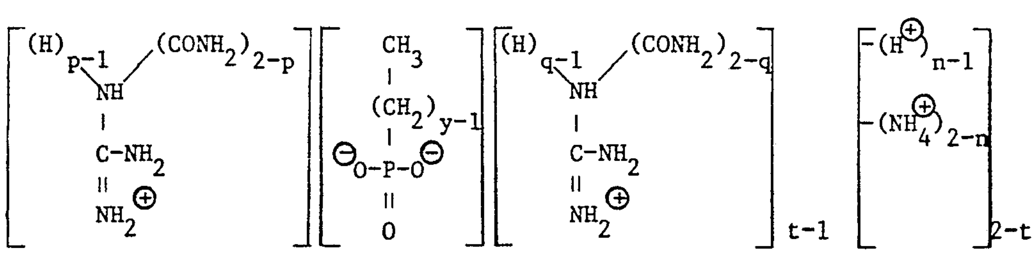

worin n, p, q, t und y je 1 oder 2 sind.

2. Phosphonsäuresalze nach Anspruch 1, dadurch gekennzeichnet, dass sie der Formel

(Siehe Figur Seite 18 f.)

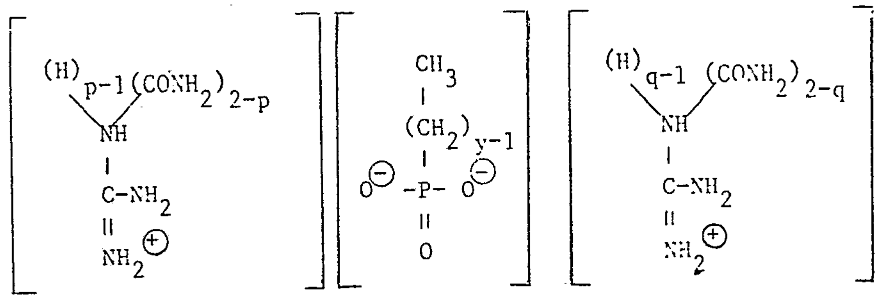

entsprechen, worin p, q und y je 1 oder 2 sind.

3. Phosphonsäuresalze nach Anspruch 2, dadurch gekennzeichnet, dass sie einer der Formeln

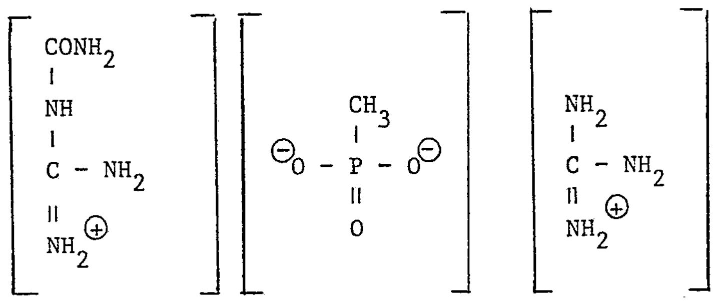

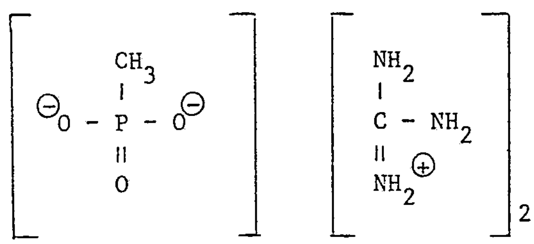

oder

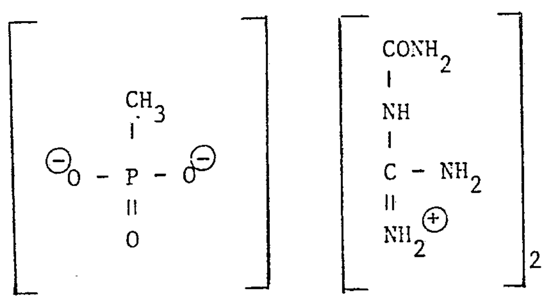

entsprechen.

4. Phosphonsäuresalze nach Anspruch 1, dadurch gekennzeichnet, dass sie der Formel

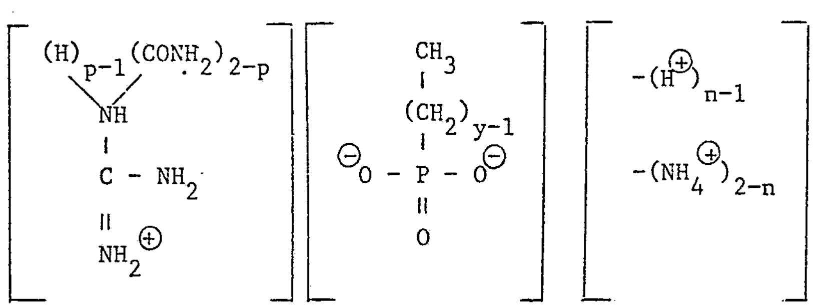

entsprechen, worin n, p und y je 1 oder 2 sind.

5. Phosphonsäuresalze nach Anspruch 4, dadurch gekennzeichnet, dass sie der Formel

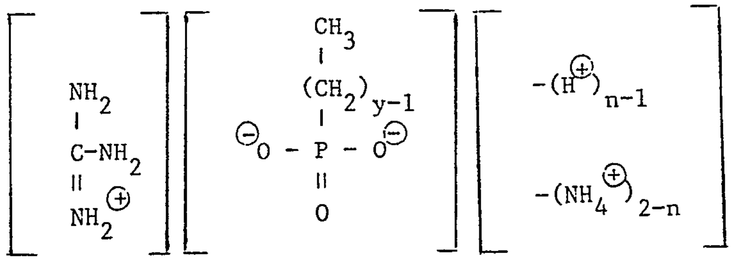

entsprechen, worin n und y je 1 oder 2 sind.

6. Phosphonsäuresalze nach Anspruch 5, dadurch gekennzeichnet, dass sie einer der Formeln

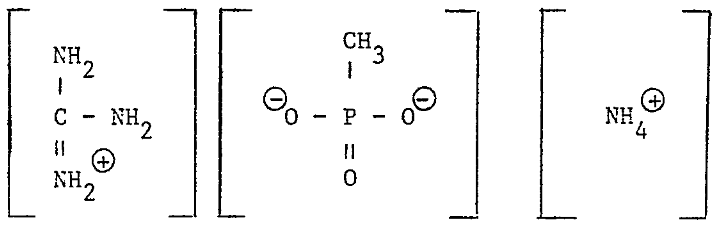

oder

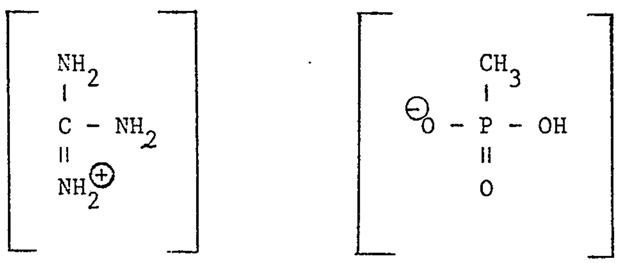

entsprechen.

7. Phosphonsäuresalze nach Anspruch 4, dadurch gekennzeichnet, dass sie der Formel

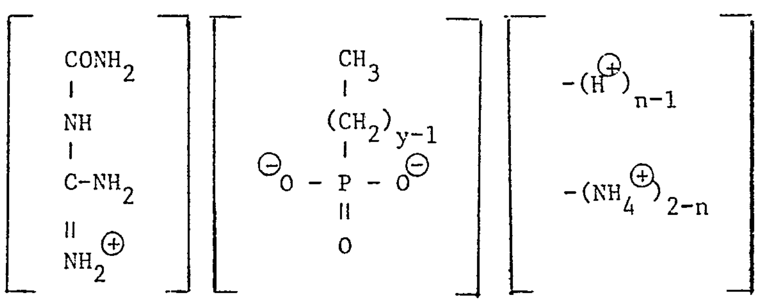

entsprechen, worin n und y je 1 oder 2 sind.

8. Phosphonsäuresalze nach Anspruch 7, dadurch gekennzeichnet, dass sie einer der Formeln

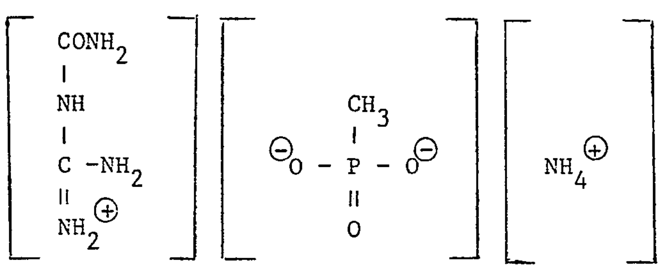

oder

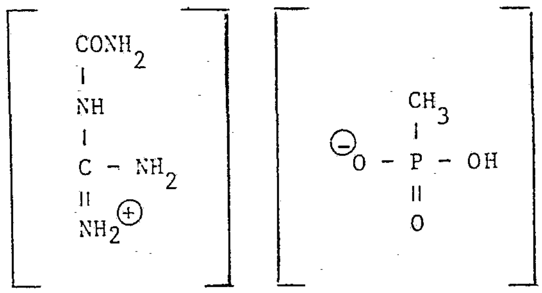

entsprechen.

9. Verfahren zur Herstellung der Phosphonsäuresalze der in Anspruch 1 angegebenen Formel (I), dadurch gekennzeichnet, dass man 1 Mol Phosphonsäure der Formel

(18) 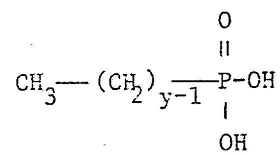

worin y 1 oder 2 ist, mit 1 Mol einer Stickstoffverbindung der Formel

(19) 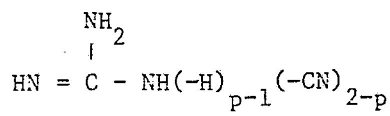

worin p 1 oder 2 ist und 1 Mol einer Stickstoffverbindung der Formel

(20) 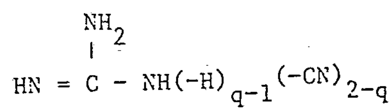

worin q 1 oder 2 ist, sofern t in der Formel (I) 2 ist, oder mit 1 Mol einer Stickstoffverbindung der Formel (19), worin p 1 oder 2 ist und gegebenenfalls 1 Mol Ammoniak, sofern t in Formel (I) 1 ist, in wässrigem Medium gegebenenfalls bei erhöhter Temperatur umsetzt, wobei die Stickstoffverbindung als freies Guanidin oder als Guanidincarbonat vorliegt, sofern p und q 2 sind und die Umsetzung unter Anlagerung von 1 Mol Wasser erfolgt, sofern p und q 1 sind und unter Abspaltung von 1 Mol Kohlendioxyd erfolgt, sofern Guanidincarbonat eingesetzt wird.

10. Verwendung der Phosphonsäuresalze gemäss einem der Ansprüche 1 bis 8 als Flammschutzmittel für organisches Fasermaterial.

**Claims**

1. Phosphonic acid salts of the formula

(1) 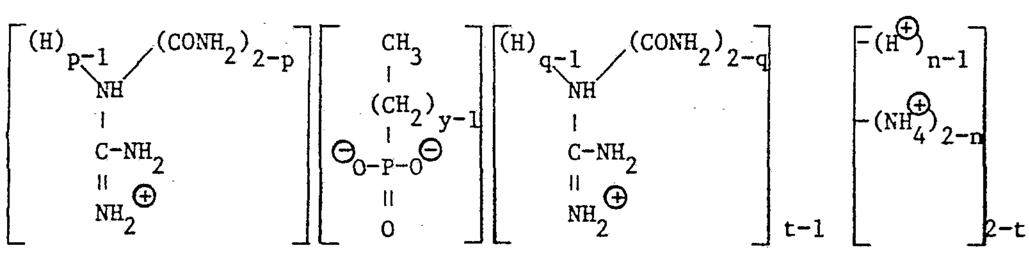

wherein n, p, q, t and y are each 1 or 2.

2. Phosphonic acid salts according to Claim 1, which correspond to the formula

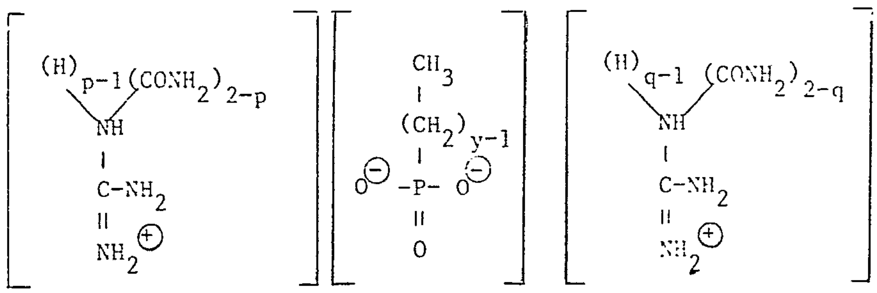

wherein p, q and y are each 1 or 2.

3. Phosphonic acid salts according to Claim 2, which correspond to any one of the formulae

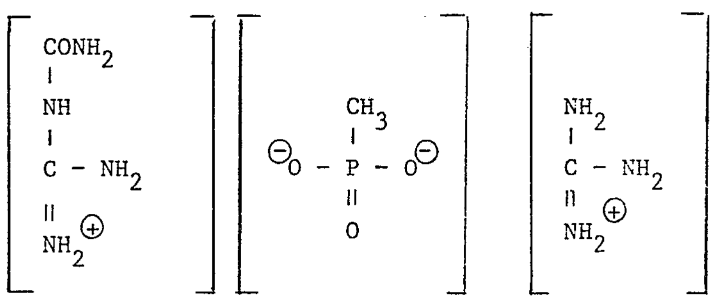

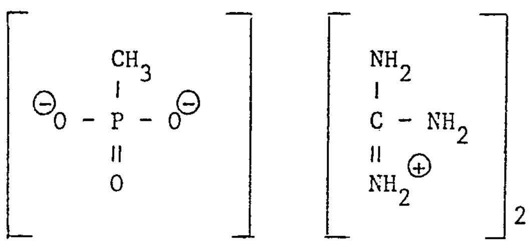

or

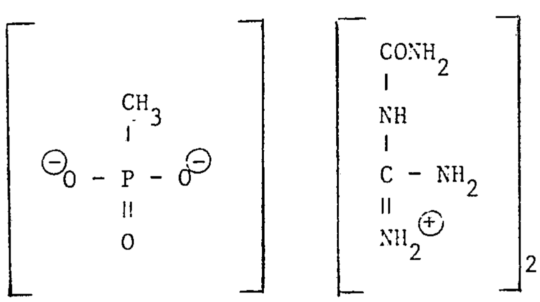

4. Phosphonic acid salts according to Claim 1, which correspond to the formula

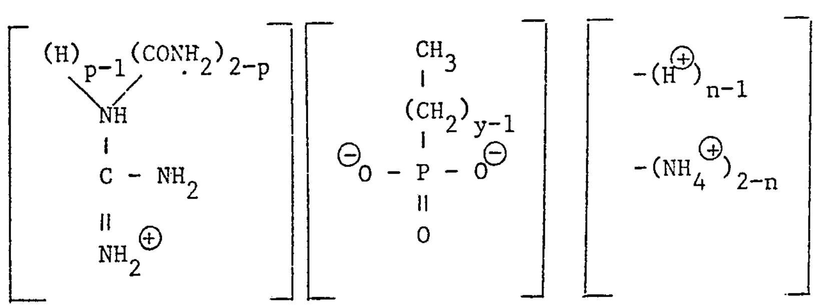

wherein n, p and y are each 1 or 2.

5. Phosphonic acid salts according to Claim 4, which correspond to the formula

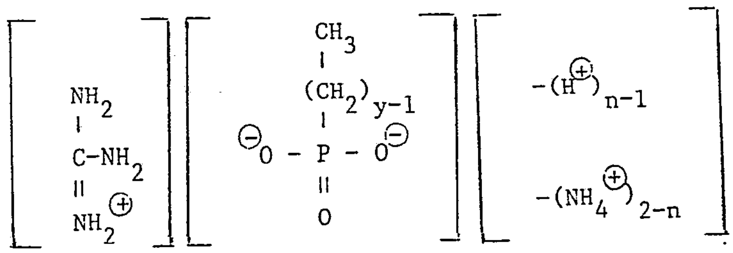

wherein n and y are each 1 or 2.

6. Phosphonic acid salts according to Claim 5, which correspond to any one of the formulae

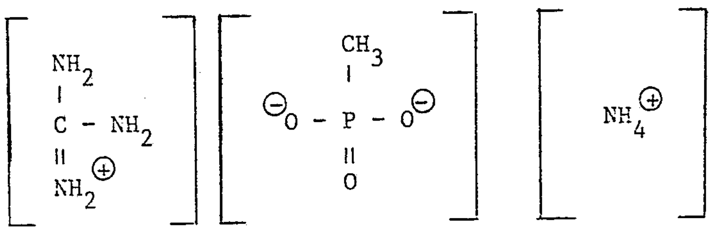

or

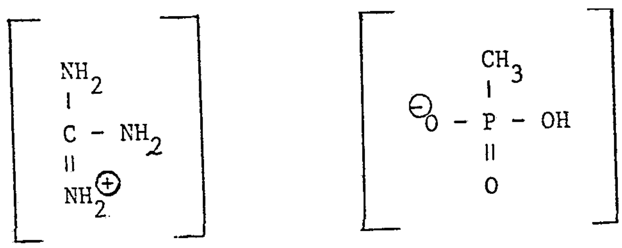

7. Phosphonic acid salts according to Claim 4, which correspond to the formula

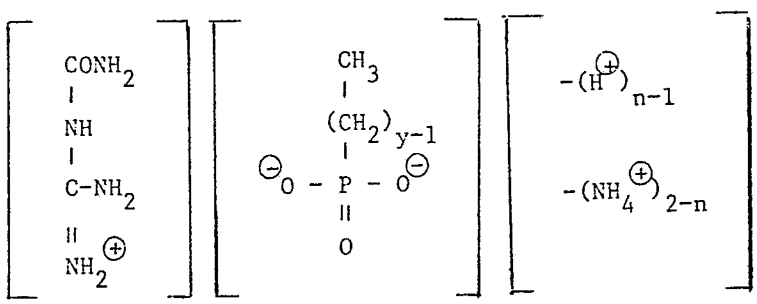

wherein n and y are each 1 or 2.

8. Phosphonic acid salts according to Claim 7, which correspond to either of the formulae

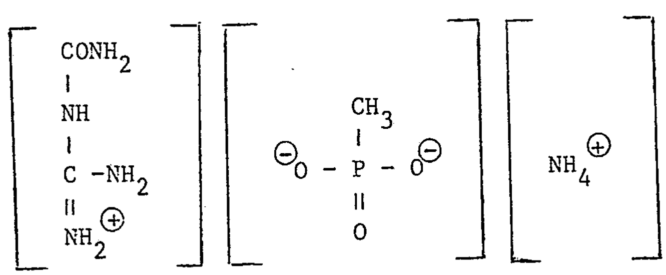

and

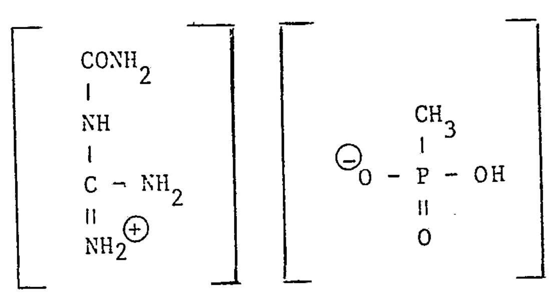

9. A process for producing the phosphonic acid salts of the formula (1) given in Claim 1, which process comprises reacting in an aqueous medium, optionally at elevated temperature, 1 mol of phosphonic acid of the formula

(18)

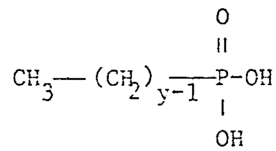

wherein y is 1 or 2, with 1 mol of a nitrogen compound of the formula

(19)

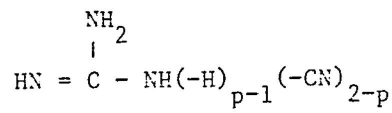

wherein p is 1 or 2, and 1 mol of a nitrogen compound of the formula

(20)

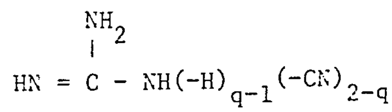

wherein q is 1 or 2, when t in the formula (1) is 2, or with 1 mol of a nitrogen compound of the formula (19) wherein p is 1 or 2, and optionally 1 mol of ammonia when t in the formula (1) is 1, the nitrogen compound being present as free guanidine, or as guanidine carbonate, when p and q are each 2, and the reaction being performed with the addition of 1 mol of water when p and q are each 1, and being performed with the removal of 1 mol of carbon dioxide when guanidine carbonate is used.

10. Use of the phosphonic acid salts according to any one of Claims 1 to 8 as fireproofing agents for organic fibre material.

**Revendications**

1. Sels d'acides phosphoniques de formule

(1)

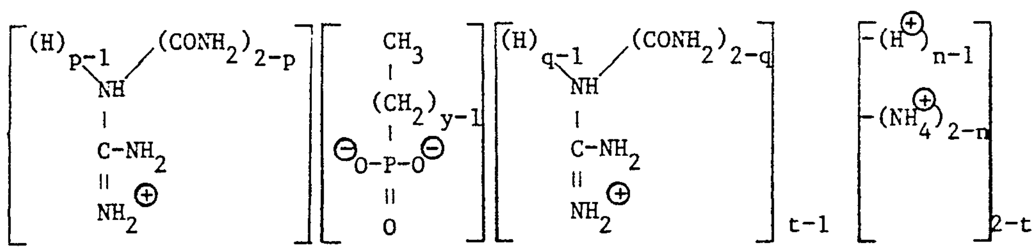

dans laquelle n, p, q, t et y valent chacun 1 ou 2.

2. Sels d'acides phosphoniques selon la revendication 1, caractérisés en ce qu'ils correspondent à la formule

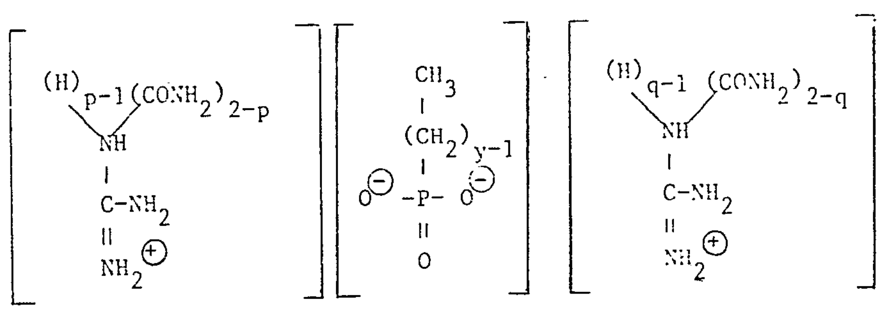

dans laquelle p, q et y valent chacun 1 ou 2.

3. Sels d'acides phosphoniques selon la revendication 2, caractérisés en ce qu'ils correspondent à l'une des formules

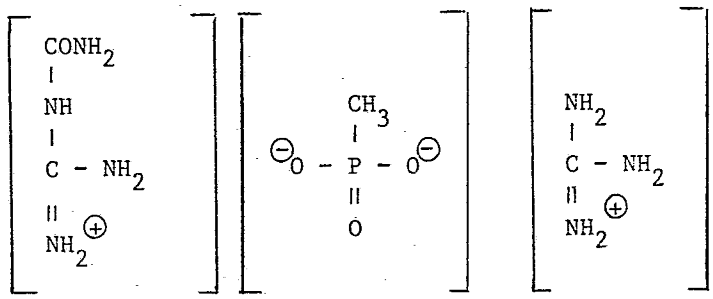

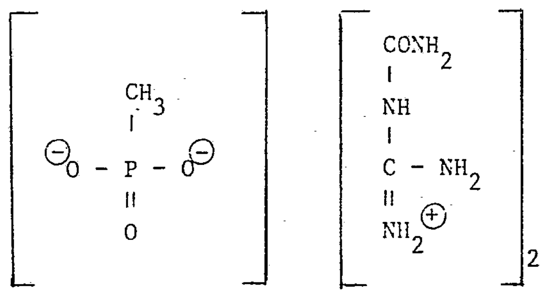

ou

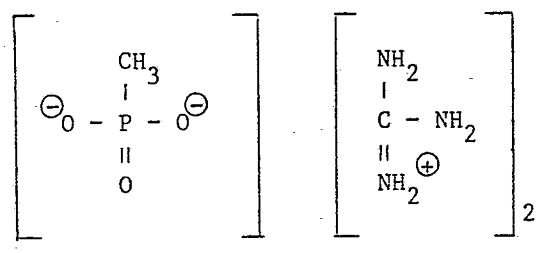

4. Sels d'acides phosphoniques selon la revendication 1, caractérisés en ce qu'ils correspondent à la formule

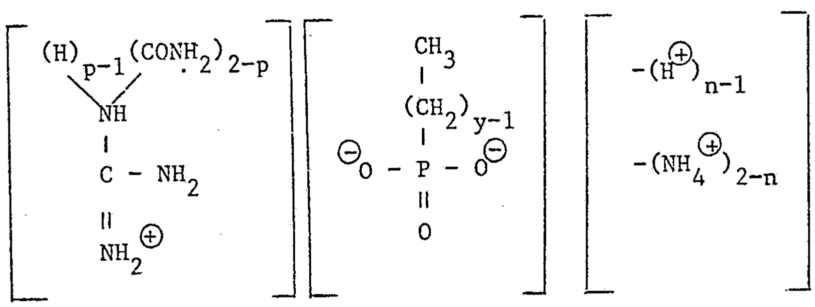

dans laquelle n, p et y valent chacun 1 ou 2.

5. Sels d'acides phosphoniques selon la revendication 4, caractérisés en ce qu'ils correspondent à la formule

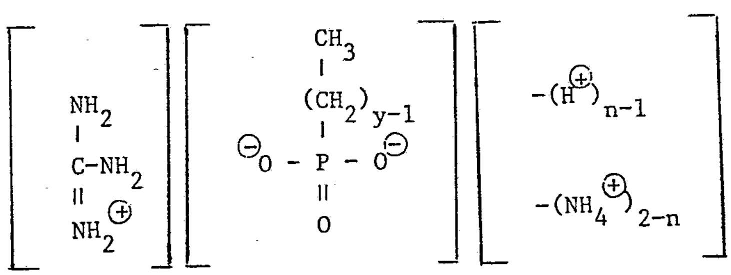

dans laquelle n et y valent chacun 1 ou 2.

6. Sels d'acides phosphoniques selon la revendication 5, caractérisés en ce qu'ils correspondent à l'une des formules

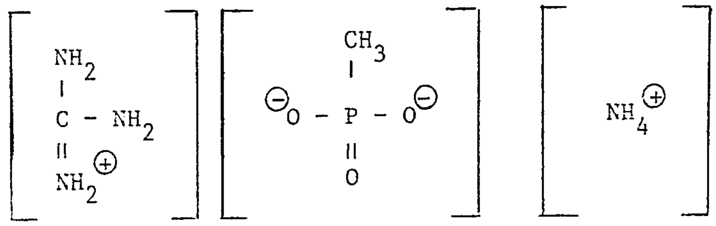

ou

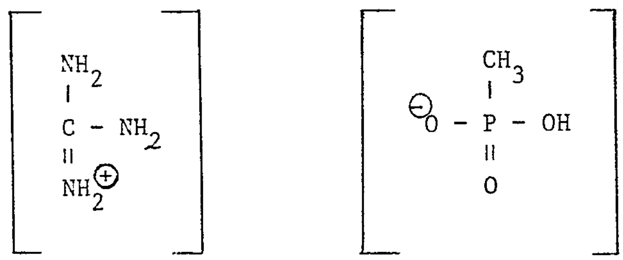

7. Sels d'acides phosphoniques selon la revendication 4, caractérisés en ce qu'ils correspondent à la formule

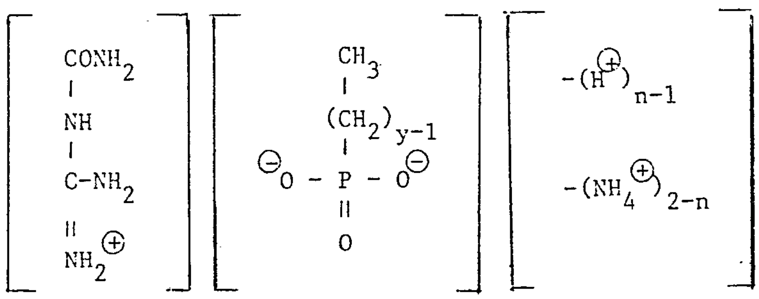

dans laquelle n et y valent chacun 1 ou 2.

8. Sels d'acides phosphoniques selon la revendication 7, caractérisés en ce qu'ils correspondent à l'une des formules

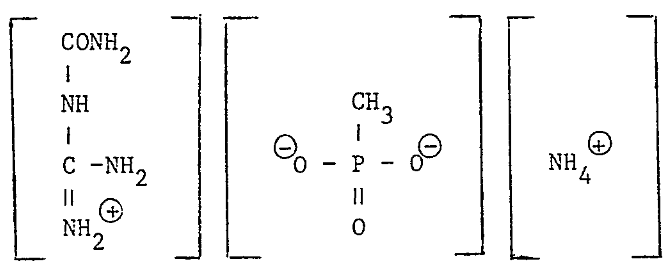

ou

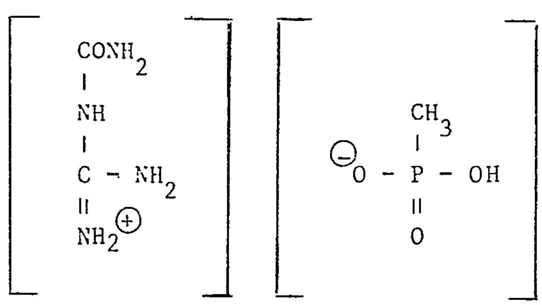

9. Procédé de préparation de sels d'acides phosphoniques de formule (1) indiquée dans la

revendication 1, caractérisé en ce qu'on condense 1 mole d'acide phosphonique de formule

(18) 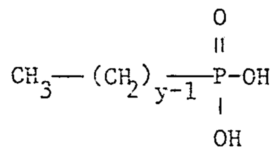

dans laquelle y vaut 1 ou 2, avec 1 mole d'un composé azoté de formule

(19) 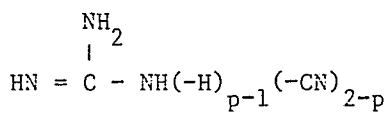

dans laquelle p vaut 1 ou 2 et avec une mole d'un composé azoté de formule

(20) 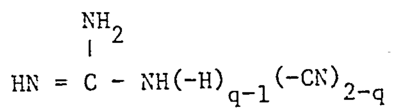

dans laquelle q vaut 1 ou 2, dans la mesure où t dans la formule (1) vaut 2, ou avec une mole de composé azoté de formule (19) dans laquelle p vaut 1 ou 2, et avec le cas échéant 1 mole d'ammoniac, dans la mesure où t vaut 1 dans la formule (1), en milieu aqueux, le cas échéant à haute température, dans lequel le composé azoté se présente sous forme de guanidine libre ou de carbonate de guanidine, dans la mesure où p et q valent 2, et la condensation se fait avec adjonction de 1 mole d'eau, dans la mesure où p et q valent 1, et par dégagement de 1 mole de gaz carbonique, dans la mesure où on utilise du carbonate de guanidine.

10. Utilisation des sels d'acides phosphoniques selon l'une des revendications de 1 à 8 comme agent ignifugeant pour matériau de fibres organiques.